# EUROPEAN PATENT SPECIFICATION

(11) **EP 2 407 147 B1**
(45) Date of publication and mention of the grant of the patent: **11.04.2018**
(21) Application number: 11173972.8
(22) Date of filing: 14.07.2011
(51) Int. Cl.: A61K 8/73, A61K 8/96, A61L 26/00, A61P 17/00

(54) **Composition with bio-regenerative, restorative and eutrophying activity**
Zusammensetzung mit biologisch regenerativer, restaurativer und eutrophierender Aktivität
Composition dotée d'une activité biorégénératrice, rétablissante et eutrophisante

(30) Priority: 14.07.2010 IT TO20100611
(43) Date of publication of application: 18.01.2012
(73) Proprietor: Mastelli S.r.l., 18038 Sanremo (IM) (IT)
(72) Inventor: Cattarini Mastelli, Giulia, I-16122 Genova (IT); Cattarini Mastelli, Laura, I-18018 Taggia (Imperia) (IT)
(74) Representative: Rambelli, Paolo

(56) References cited:
- WO-A1-2005/051446
- WO-A1-2010/049898
- IORIZZO M ET AL: "Biorejuvenation: theory and practice", CLINICS IN DERMATOLOGY, J.B. LIPPINCOTT, PHILADELPHIA, PA, US, vol. 26, no. 2, 1 March 2008 (2008-03-01), pages 177-181, XP022647617, ISSN: 0738-081X, DOI: DOI:10.1016/J.CLINDERMATOL.2007.09.011 [retrieved on 2008-05-08] & LINDAHL T ET AL: "RATE OF DEPURINATION OF NATIVE DEOXYRIBONUCLEIC ACID", BIOCHEMISTRY, AMERICAN CHEMICAL SOCIETY, US, vol. 11, no. 19, 1 January 1972 (1972-01-01), pages 3610-3618, XP002379056, ISSN: 0006-2960, DOI: 10.1021/BI00769A018
- PATTERSON JENNIFER ET AL: "Hyaluronic acid hydrogels with controlled degradation properties for oriented bone regeneration", BIOMATERIALS, vol. 31, no. 26, 23 June 2010 (2010-06-23) , pages 6772-6781, XP002630553, ISSN: 0142-9612
- VALDATTA L ET AL: "Evaluation of the efficacy of polydeoxyribonucleotides in the healing process of autologous skin graft donor sites: A pilot study", CURRENT MEDICAL RESEARCH AND OPINION, INFORMA HEALTHCARE, GB, vol. 20, no. 3, 1 March 2004 (2004-03-01), pages 403-408, XP008122537, ISSN: 0300-7995
- TONELLO GIUSEPPE ET AL: "Characterization and quantitation of the active polynucleotide fraction (PDRN) from human placenta, a tissue repair stimulating agent", JOURNAL OF PHARMACEUTICAL AND BIOMEDICAL ANALYSIS, NEW YORK, NY, US, vol. 14, no. 11, 1 January 1996 (1996-01-01), pages 1555-1560, XP002583526, ISSN: 0731-7085
- CHEN W Y J ET AL: "FUNCTIONS OF HYALURONAN IN WOUND REPAIR", WOUND REPAIR AND REGENERATION, MOSBY-YEAR BOOK, ST. LOUIS, MO, US, vol. 7, no. 2, 1 March 1999 (1999-03-01), pages 79-89, XP008005341, ISSN: 1067-1927, DOI: DOI:10.1046/J.1524-475X.1999.00079.X

## Description

This disclosure relates to a composition comprising polynucleotides (PN) and hyaluronic acid having scar-forming, re-epithelialising, bioregenerative and eutrophicating activity for a variety of connective and/or epithelial tissues, including tissues of the skin, bone, joints, osteotendinous and myotendinous junctions, mucosa and structures of the visual system, and is therefore useful in the treatment of diseases affecting these tissues, and in cosmetics.

Bioregeneration and wound repair have common physiopathological bases which conventionally progress through the sequence:
1. inflammatory stage
2. proliferative stage
3. remodelling stage.

Biologically, these formally separate and sequential stages often overlap substantially. We can say that in these processes the herein described composition acts to encourage the initial stages primarily but not exclusively through the hyaluronic acid, and the final stages primarily but not exclusively through the polynucleotides (PN).

During the inflammatory stage there is a progressive reaction by cytokines which induce the release of large molecule (thousands of kDalton) hyaluronic acid which has a hydrating function and facilitates cell migration, that then breaks up into fragments through its marked pro-inflammatory role facilitating the first stages of repair. The function of the hyaluronic acid is therefore characteristic of the first stage of the inflammatory process and precedes proper development of the wound-healing process.

Cell migration by fibroblasts and neoangiogenesis are central phenomena in completion of the first stage.

During the proliferative stage the fibroblasts regenerate and produce an extracellular matrix. Hyaluronic acid is no longer needed during this stage and the "filling" role is performed by all the components of the skin and other tissues (collagen, GAG, proteins, glycoproteins, etc.) secreted by the cells specific to the tissue involved. This stage is mainly controlled by polynucleotides.

Iorizzo M. et al., Clinics in Dermatology, J. B. Lippincott, Philadelphia, PA, US, Vol. 26, No. 2, 1 March 2008, pages 177-181, describes theory and practice of biorejuvenation and mentions, as products available in the mesotherapy for skin rejuvenation polynucleotide macromolecules, hyaluronic acid alone and hyaluronic acid plus other active ingredients including vitamins, amino acids, minerals, coenzymes, nucleic acids, beta-glucan.

WO 2005/051446 discloses the use of hyaluronic acids in a composition for bone tissue regeneration.

Patterson et al., Biomaterials, Vol. 31, No. 26, 23 June 2010, pages 6772-6781, discloses the use of hyaluronic acid hydrogels for bone tissue regeneration.

WO 2010/049898 discloses the use of polynucleotides for treating osteoarticular diseases.

This invention is based on recognition of the fact that the joint administration of hyaluronic acid and PN has a positive effect during all stages of regeneration, eutrophication and tissue repair, demonstrating synergy between the components which makes it possible to achieve better results than with administration of the components used separately.

Thus an object of the invention is a composition having a synergistic effect comprising polynucleotides and hyaluronic acid for the use defined by the following claims, which form an integral part of the description, said composition being defined in said claims.

Hyaluronic acid (HA) is the only non-sulphur containing polymer belonging to the family of glycosaminoglycanes; it is ubiquitous in mammalian tissue, does not have any branches in its structure and comprises an orderly repetition of a disaccharide comprising glucuronic acid and N-acetyl-glucosamine.

The size of the HA varies, but exceeds one million Daltons, varying between 3 and 9 million in some tissues. Initially it was isolated by Mayer in 1934 from the hyaloid substance of the vitreous body, whence its name, and it has been recognised for many years that HA is a filling and structural substance having extraordinary hydrophilic capabilities without a particular biological role. It is only during the last decade that the biological role of this polymer has been identified, particularly following the discovery of tissue receptors for HA such as CD44 and RHAMM. Knowledge of the role of HA as an active substance in more complex processes relating to tissue regeneration and repair has therefore grown.

Organisation of the data available in the literature has therefore introduced a new concept in which HA is no longer considered to be an inert molecule, but actively involved in important biological processes such as cell migration and division, angiogenesis and tissue growth and maturation.

The functions of HA vary significantly depending upon its size, sometimes producing amazing effects. In fact high molecular weight molecules are highly hydrating and facilitate cell migration, while smaller fragments have an action encouraging inflammatory processes. These are phenomena involved in the initial stages of regenerative and reparative processes.

A. Within the scope of this invention hyaluronic acid may be used as such or as various salts, for example with sodium.

Typically, hyaluronic acid or its sodium salt having a molecular weight of between 500 and 3000 kDalton, preferably between 600 and 1600 kDalton, are used.

B. The term "Polynucleotides" is taken to indicate a fraction of a polymer chain of nucleic acid, preferably DNA, having a molecular weight distribution preferably within the range between 0.5 kDalton and 3000 kDalton, more preferably between 30 kDalton and 2000 kDalton, having a numerical mean molecular weight of for example approximately 250 kDalton. This fraction of DNA polymer chains is obtained by extraction from fish sperm or from a plant source, through a process which comprises a stage of at least partial depurination of the DNA to eliminate its information-bearing capacity so as to obtain a mixture of polynucleotides having a percentage depurination of between 1% and 5% of purine bases removed out of the total purine bases originally present and preferably a stage of partial fragmentation of the DNA chains in order to reduce their molecular weights.

The percentage depurination of DNA lies within the range of 1% to 5% of purine bases removed (or purine-free sites) out of the total of purine bases originally present. A preferred percentage depurination is between 2% and 2.5%.

From what has been stated above it is obvious that the characteristics of the PN are appreciably different from those of conventional polynucleotides which have not been subjected to:
- partial depurination,
- sterilisation techniques for viruses and prions,
- procedures for purification of the product which when dry exceeds 98% purity with quantities of proteins below 0.5% and a molecular weight range varying between 0.2 and 3000 kDalton.

It is known and documented that PN have the ability to induce mitosis in fibroblasts, endothelial cells and other cells. They have been used to stimulate tissue repair and in many experimental models have shown appreciable capacity for the ex novo synthesis of molecules specific to the extracellular matrix.

In all wound-healing processes the scientific literature has confirmed that PN have a positive effect on damage repair. PN also have an appreciable ability to modulate the action of many inflammatory factors and to interact with physiological growth factors, which make them very important and biologically very active molecules in the growth of different cell lines and their secretory activity.

The polynucleotides used have a molecular weight distribution preferably within the range from 0.2 kDalton to 3000 kDalton, more preferably between 30 kDalton and 2000 kDalton, with for example a mean numerical molecular weight of approximately 250 kDalton.

The composition comprises:
- hyaluronic acid as described above
- polynucleotides (PN) as described above.

For liquid formulations (for example injectable formulations or collyria) polyols are used to obtain suitable osmolality, preferably mannitol in concentrations from 0 mg/ml to 50 mg/ml, while to ensure a pH of around neutrality (7.0 +/- 1.0) a buffer system, preferably phosphate buffer, is used.

The mannitol used is sterile, apyrogenic, or pharmaceutical grade, for injectable use according to the European Pharmacopoeia.

The phosphate buffer used is of pharmaceutical grade, for injectable use, sterile and apyrogenic.

The compositions used typically comprise:
- polynucleotides from 1 mg/ml to 20 mg/ml, preferably from 5 mg/ml to 12 mg/ml,
- hyaluronic acid from 1 mg/ml to 20 mg/ml, preferably from 5 mg/ml to 12 mg/ml with a polynucleotides/hyaluronic acid ratio by weight within the range from 0.2:1 to 2:1.

### Example 1

| | |
|---|---|
| Mannitol: | 40 mg/ml |
| phosphate buffer: | 1.3 mg/ml |
| hyaluronic acid: | 7.5 mg/ml |
| PN: | 7.5 mg/ml |

### Example 2

| | |
|---|---|
| Mannitol: | 40 mg/ml |
| phosphate buffer: | 1.3 mg/ml |
| hyaluronic acid: | 10 mg/ml |
| PN | 10 mg/ml |

### Example 3

| | |
|---|---|
| Mannitol: | 40 mg/ml |
| phosphate buffer: | 1.3 mg/ml |
| hyaluronic acid: | 3.5 mg/ml |
| PN | 3.5 mg/ml |

### Example 4

| | |
|---|---|
| Mannitol: | 40 mg/ml |
| phosphate buffer: | 1.3 mg/ml |
| hyaluronic acid: | 5 mg/ml |
| polynucleotides: | 15 mg/ml |

### Example 5

| | |
|---|---|
| Mannitol: | 40 mg/ml |
| phosphate buffer: | 1.3 mg/ml |
| hyaluronic acid: | 3 mg/ml |
| PN (respectively): | 7.5, 11, 16 mg/ml |

The synergistic effect of the compositions according to the invention has been determined through in vitro tests to determine the effect of pure hyaluronic acid, pure PN and a combination of the two on cell growth and protein expression.

### Description of the tests

Fibroblasts of human origin were cultured on growth medium (Dulbecco's Modified Eagle Medium) supplemented with 10% bovine foetal serum (BFS). The cells were seeded with a density of 10,000/cm². 24 hours after seeding the cells were incubated under the conditions shown in the table, still prepared in DMEM + 10% BFS.

Cells were counted and proteins were quantified after 3 days' treatment. The number of cells was measured using a cell counting instrument, proteins were quantified from the spectrophotometric reading of stained protein lysates using a modified Lowry's method.

The table below shows the concentrations of the compositions tested. The values relating to cell growth and protein content are expressed as % of the control, and the latter should be regarded as being a culture of fibroblasts not subjected to any treatment, measured at the same point in time.

**Table 1**

| **COMPOSITION** | **CONCENTRATION (µg/ml)** | **CELL GROWTH (% in comparison with control)** | **PROTEIN EXPRESSION (% in comparison with control)** |
|---|---|---|---|
| Control: | | 100 | 100 |
| Hyaluronic acid (HA) | 12 | 104 | 103 |
| | 37 | 104 | 105 |
| | 50 | 103 | 104 |
| | 111 | 105 | 104 |
| | 333 | 106 | 107 |
| PN | 12 | 117 | 115 |
| | 37 | 121 | 119 |
| | 50 | 125 | 126 |
| | 111 | 127 | 127 |
| | 333 | 129 | 128 |
| HA + PN | 6 + 6 | 112 | 113 |
| | 18.5 + 18.5 | 126 | 125 |
| | 25 + 25 | 128 | 128 |
| | 55.5 + 55.5 | 130 | 131 |
| | 166.5 + 166.5 | 133 | 131 |

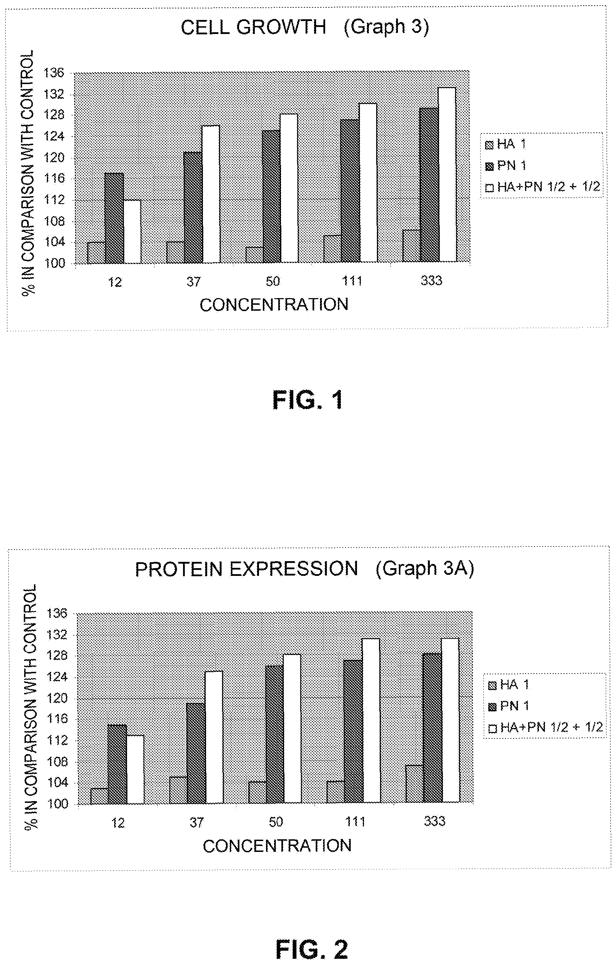

The data in Table 1 and the corresponding graphs in Figure 1 and Figure 2 show that hyaluronic acid does not change its action as its concentration increases, constantly maintaining a mild effect both as regards protein expression and cell growth. Nevertheless it is clear that the polynucleotides increase their effect with increasing dose.

With regard to the individual components, the effect obtained by the mixture - which for the same total concentration comprised 50% hyaluronic acid and 50% polynucleotides - was consistently better than the effect of the PN used alone and of course the HA used alone (e.g.: the comparison was made between 37 µg/ml of HA used alone, 37 µg/ml of PN used alone and a mixture containing 18.5 µg/ml of both); thus bearing in mind the small effect of hyaluronic acid alone it is obvious that the presence of the latter is important in increasing the effect of the PN, these being the components which have real significance for cell growth and protein expression.

It can be concluded that by combining HA and PN a synergistic effect in cell regeneration and the deposition of protein matrix is obtained.

Bearing in mind the fact that the concentration of hyaluronic acid is substantially irrelevant for the purposes of cell growth and matrix production, a further test was performed in which the concentration of hyaluronic acid was maintained constant at 1 mg/ml and the PN were used in increasing concentrations. The decision to set the HA concentration at 1 mg/ml was brought about as a result of the previous experiments in which it was found that when HA was tested alone at various dilutions, although its ability to encourage regeneration and cell secretion was relatively constant and modest, this was the maximum effective concentration under the experimental conditions.

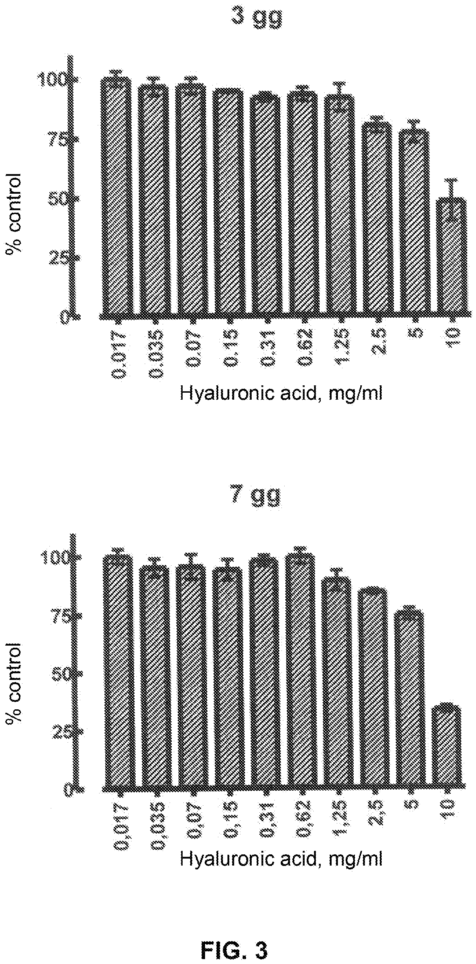

The decision to set the concentration of the Hyaluronic Acid to be used in the mixture at 1 mg/ml was determined by the data obtained by testing different dilutions of Hyaluronic Acid on fibroblasts in culture for 3 and 7 days. Cell growth was evaluated by the Alamar Assay method. The data are shown in the graph in Figure 3, expressed as percentage growth of the treated cells in comparison with the control, set at 100.

On the basis of these experimental data the dose of 1 mg/ml of HA was identified as being the most suitable dose in that it is the maximum which can be administered without an adverse interference on cell growth, thus making best use of the hydrating effect of the HA.

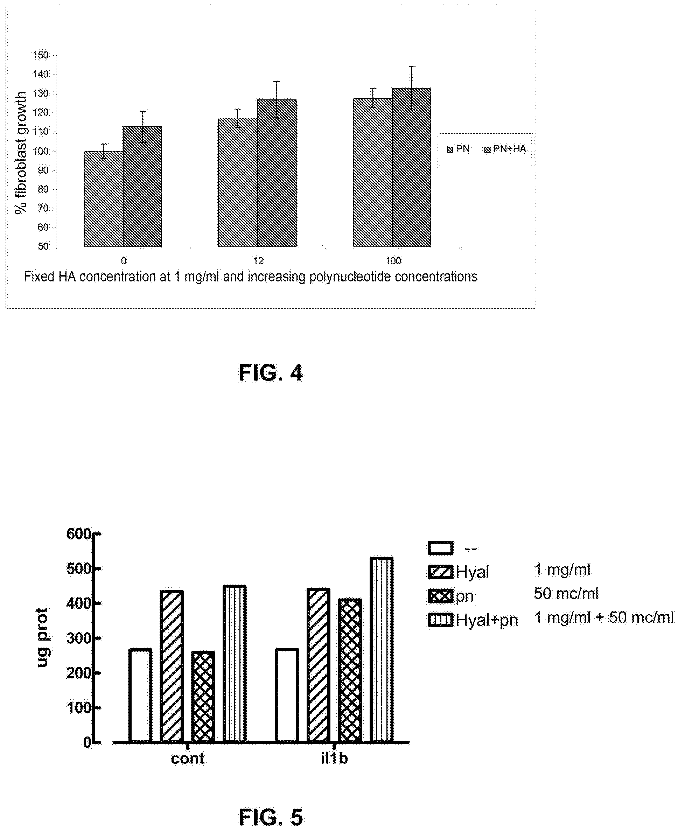

The graph in Figure 4 and corresponding Table 2 below confirm that HA significantly potentiates the effect of the PN, in fact 12 µ/ml of PN in the presence of HA yields results equivalent to 100 µg/ml in the absence of HA.

**Table 2**

| | PN | | PN+HA | | | | | | |
|---|---|---|---|---|---|---|---|---|---|
| | mean | sd | mean | sd | | min | max | min | max |
| 0 | 100 | 3.9 | 112.8 | 8.14 | | 96.1 | 103.9 | 104.66 | 120.94 |
| 12 | 117 | 4.6 | 126.82 | 9.45 | | 112.4 | 121.6 | 117.37 | 136.27 |
| 100 | 127.8 | 4.88 | 133.03 | 11.4 | | 122.92 | 132.68 | 121.63 | 144.43 |

Furthermore HA is a substance which has a particular part to play in the control of tissue hydration and this is an important advantage over and above the effect of potentiating PN, providing the mixture with an effect which is undoubtedly useful for the applications comprising the object of the invention.

Thus the combination of polynucleotides and hyaluronic acid in the same mixture has a complementary effect - the former stimulate the increase in fibroblasts and extracellular protein content, the latter encourages hydration of the matrix. A synergistic activity is therefore achieved restoring all the components of the treated tissue allowing tissue trophism to recover where necessary and lesions to be repaired where there has been loss of substance.

Further experimental tests have shown that polynucleotides (PN) have a potentiating effect on the activity of hyaluronic acid in producing proteins in synoviocytes in the presence or in the absence of a prior inflammatory stimulus due to interleukin 1 beta (IL1b).

The results of the tests are shown in the histogram in Figure 5, in which the quantity of protein in µg produced following the stimulation of synoviocytes with control, hyaluronic acid (Hyal, 1 mg/ml), polynucleotides (PN, 50 µ/ml) and Hyal + PN (1 mg/ml and 50 µ/ml) in the presence and absence (cont) of the inflammatory stimulus IL1b is plotted as the ordinate.

Whereas the subject-matter of the invention relates to the compositions for the use defined by the appended claims, in view of the results observed, the described compositions may be used in all applications of a therapeutic or cosmetic nature in which the following activities are required:
a) activity to bioregenerate the skin, for example on the face, neck, breast, skin in general, skin folds and mucosal skin (major and minor labia) and cutaneous annexes, such as the scalp for the biorevitalisation of hair;
b) trophic, reparative and lubricant activities, both when applied topically (skin lesions or dystrophies), or when administered through intra-articular instillation;
c) trophic and reparative activity through perilesional instillation in the case of ulcers, bedridden patients and loss of substance of different kinds;
d) activity in re-epithelialisation of the conjunctiva and cornea, when topically administered through collyria, creams or ophthalmic gels.

Suitable pharmaceutical forms for the applications mentioned above may be:
- multi-use bottles, single-use vials, pre-filled and disposable syringes,
- sprays or foams, medicated gauzes or other media for topical applications,
- liquids or emulsions or other forms for transdermal administration through suitable devices,
- mixtures with suitable excipients for formulations which can be administered to mucosal surfaces, natural orifices (oral cavity, vagina, rectum): ovuli, suppositories, tablets and pessaries, cannulas, ointments, creams, gels,
- ophthalmic drops, as tear substitutes and/or having a re-epithelialising effect,
- emulsions (creams, ointments, sera, gels) of different types for topical applications and for aesthetic/regenerative purposes, as well as activities encouraging the repair of loss of substance, intended for the epithelia, mucosa and connective tissues, including ocular structures.

## Claims

1. Composition comprising hyaluronic acid and a mixture of polynucleotides extracted from fish sperm or from a plant source, the said mixture of polynucleotides being a fraction of the polymer chains of at least partly depurinated DNA in which the said mixture of polynucleotides has a percentage depurination of between 1% and 5% of purine bases removed out of the total purine bases originally present and has a molecular weight distribution between 0.5 kDalton and 3000 kDalton for use in therapeutic treatment for the regeneration and/or repair of skin tissues.

2. A composition for use according to Claim 1 for therapeutic bioregeneration treatment of the skin and scalp.

3. Composition for use according to Claim 1 for use in treatments for the repair of cutaneous tissue through subcutaneous instillation.

4. Composition for use according to Claim 1 for cutaneous applications in formulations suitable for transdermal transport.

5. Composition for use according to any one of Claims 1 to 4 comprising 1 mg/ml to 20 mg/ml of hyaluronic acid and 1 mg/ml to 20 mg/ml of the said mixture of polynucleotides.

6. Composition for use according to Claim 5, comprising 5 mg/ml to 12 mg/ml of hyaluronic acid and 5 mg/ml to 12 mg/ml of the said mixture of polynucleotides.

7. Composition for use according to Claims 5 or 6, comprising polynucleotides and hyaluronic acid in a ratio by weight of between 0.2:1 and 2:1.

8. Composition for use according to any one of the preceding claims, in which the said mixture of polynucleotides is a fraction of the polymer chains of nucleic acid having a molecular weight distribution between 30 kDalton and 2000 kDalton.

9. Composition for use according to any one of the preceding claims, in which the said mixture of polynucleotides is obtained by extraction from fish sperm or from a plant source by a procedure comprising a stage of partial fragmentation of the polynucleotide chains of nucleic acid and a stage of partial depurination of the nucleic acid.

10. Composition for use according to any one of the preceding claims, comprising hyaluronic acid having a molecular weight of between 500 kDalton and 3000 kDalton.

11. Composition for use according to any one of the preceding claims in liquid form, not containing physiological saline solution, in an aqueous solvent comprising a pharmaceutically-acceptable polyol, preferably mannitol.

12. Composition for use according to Claim 11, having an osmolality of between 10 m.o.s./l and 500 m.o.s./l, preferably between 250 m.o.s./l and 350 m.o.s./l.

13. The use of a composition comprising hyaluronic acid and a mixture of polynucleotides as described in any of claims 1 and 5 to 12 for the cosmetic treatment of skin tissues.

## Patentansprüche

1. Zusammensetzung, die Hyaluronsäure und ein Gemisch von Polynucleotiden aufweist, die aus Fischsperma oder aus einer pflanzlichen Quelle extrahiert sind, wobei das Gemisch von Polynucleotiden eine Fraktion der Polymerketten einer zumindest teilweise depurinierten DNA ist, wobei das Gemisch von Polynucleotiden eine prozentuale Depurinierung aufweist, bei der zwischen 1 % und 5 % der Purinbasen aus den ursprünglich vorhandenen gesamten Purinbasen entfernt sind, und eine Molekulargewichtsverteilung zwischen 0,5 kDalton und 3000 kDalton aufweist, zur Verwendung bei einer therapeutischen Behandlung zur Regeneration und/oder Reparatur von Hautgeweben.

2. Zusammensetzung zur Verwendung gemäß Anspruch 1 zur therapeutischen Bioregenerationsbehandlung der Haut und Kopfhaut.

3. Zusammensetzung zur Verwendung gemäß Anspruch 1 zur Verwendung bei Behandlungen für die Reparatur von Kutangewebe durch subkutane Instillation.

4. Zusammensetzung zur Verwendung gemäß Anspruch 1 für kutane Anwendungen in Formulierungen, die für eine transdermale Verabreichung geeignet sind.

5. Zusammensetzung zur Verwendung gemäß einem der Ansprüche 1 bis 4, die 1 mg/ml bis 20 mg/ml Hyaluronsäure und 1 mg/ml bis 20 mg/ml des Gemischs von Polynucleotiden aufweist.

6. Zusammensetzung zur Verwendung gemäß Anspruch 5, die 5 mg/ml bis 12 mg/ml Hyaluronsäure und 5 mg/ml bis 12 mg/ml des Gemischs von Polynucleotiden aufweist.

7. Zusammensetzung zur Verwendung gemäß Anspruch 5 oder 6, die Polynucleotide und Hyaluronsäure in einem Gewichtsverhältnis zwischen 0,2:1 und 2:1 aufweist.

8. Zusammensetzung zur Verwendung gemäß einem der vorhergehenden Ansprüche, bei der das Gemisch von Polynucleotiden eine Fraktion der Polymerketten einer Nucleinsäure mit einer Molekulargewichtsverteilung zwischen 30 kDalton und 2000 kDalton ist.

9. Zusammensetzung zur Verwendung gemäß einem der vorhergehenden Ansprüche, bei der das Gemisch von Polynucleotiden durch Extraktion aus Fischsperma oder aus einer pflanzlichen Quelle mittels eines Verfahrens erhalten wird, das eine Stufe einer partiellen Fragmentierung der Polynucleotidketten einer Nucleinsäure und eine Stufe einer partiellen Depurinierung der Nucleinsäure aufweist.

10. Zusammensetzung zur Verwendung gemäß einem der vorhergehenden Ansprüche, die Hyaluronsäure mit einem Molekulargewicht zwischen 500 kDalton und 3000 kDalton aufweist.

11. Zusammensetzung zur Verwendung gemäß einem der vorhergehenden Ansprüche in flüssiger Form, die keine physiologische Kochsalzlösung enthält, in einem wässrigen Lösungsmittel, das ein pharmazeutisch akzeptables Polyol, vorzugsweise Mannitol, aufweist.

12. Zusammensetzung zur Verwendung gemäß Anspruch 11, die eine Osmolalität zwischen 10 m.o.s./l und 500 m.o.s./l, vorzugsweise zwischen 250 m.o.s./l und 350 m.o.s./l, aufweist.

13. Die Verwendung einer Zusammensetzung, die Hyaluronsäure und ein Gemisch von Polynucleotiden aufweist, wie in einem der Ansprüche 1 und 5 und 12 beschrieben ist, für die kosmetische Behandlung von Hautgeweben.

## Revendications

1. Composition comprenant un acide hyaluronique et un mélange de polynucléotides extraits à partir de sperme de poisson ou à partir d'une source végétale, ledit mélange de polynucléotides étant une fraction des chaînes polymères d'un ADN au moins partiellement dépuriné dans lequel ledit mélange de polynucléotides présente un pourcentage de dépurination situé entre 1 % et 5 % de bases purine retirées du total des bases purine initialement présentes et présente une répartition de poids moléculaires située entre 0,5 kDalton et 3000 kDalton pour son utilisation dans un traitement thérapeutique pour la régénération et/ou la réparation de tissus cutanés.

2. Composition pour son utilisation selon la revendication 1 pour un traitement de biorégénération thérapeutique de la peau et du cuir chevelu.

3. Composition pour son utilisation selon la revendication 1 pour son utilisation dans des traitements pour la réparation d'un tissu cutané par l'intermédiaire d'une instillation sous-cutanée.

4. Composition pour son utilisation selon la revendication 1 pour des applications cutanées dans des formulations appropriées pour un transport transdermique.

5. Composition pour son utilisation selon l'une quelconque des revendications 1 à 4 comprenant 1 mg/ml à 20 mg/ml d'acide hyaluronique et 1 mg/ml à 20 mg/ml dudit mélange de polynucléotides.

6. Composition pour son utilisation selon la revendication 5, comprenant 5 mg/ml à 12 mg/ml d'acide hyaluronique et 5 mg/ml à 12 mg/ml dudit mélange de polynucléotides.

7. Composition pour son utilisation selon les revendications 5 ou 6, comprenant des polynucléotides et un acide hyaluronique dans un rapport en poids situé entre 0,2 : 1 et 2 : 1.

8. Composition pour son utilisation selon l'une quelconque des revendications précédentes, dans laquelle ledit mélange de polynucléotides est une fraction des chaînes polymères d'un acide nucléique présentant une répartition de poids moléculaires situé entre 30 kDalton et 2000 kDalton.

9. Composition pour son utilisation selon l'une quelconque des revendications précédentes, dans laquelle ledit mélange de polynucléotides est obtenu par extraction à partir de sperme de poisson ou à partir d'une source végétale par une procédure comprenant une étape de fragmentation partielle des chaînes polynucléotidiques d'un acide nucléique et une étape de dépurination partielle de l'acide nucléique.

10. Composition pour son utilisation selon l'une quelconque des revendications précédentes, comprenant un acide hyaluronique présentant un poids moléculaire situé entre 500 kDalton et 3000 kDalton.

11. Composition pour son utilisation selon l'une quelconque des revendications précédentes sous forme liquide, ne contenant pas une solution saline physiologique, dans un solvant aqueux comprenant un polyol pharmaceutiquement acceptable, de préférence le mannitol.

12. Composition pour son utilisation selon la revendication 11, présentant une osmolalité située entre 10 m.o.s./l et 500 m.o.s./l, de préférence entre 250 m.o.s./l et 350 m.o.s./l.

13. Utilisation d'une composition comprenant un acide hyaluronique et un mélange de polynucléotides telles que décrite dans l'une quelconque des revendications 1 et 5 à 12 pour le traitement cosmétique de tissus cutanés.
